(19) Europäisches Patentamt
European Patent Office
Office européen des brevets

(11) EP 2 251 009 B1

(12) EUROPEAN PATENT SPECIFICATION

(45) Date of publication and mention of the grant of the patent:
**16.07.2014 Bulletin 2014/29**

(21) Application number: **09714377.0**

(22) Date of filing: **27.02.2009**

(51) Int Cl.:
***A61K 31/41*** *(2006.01)* ***A61K 9/06*** *(2006.01)*
***A61K 9/08*** *(2006.01)* ***A61K 9/20*** *(2006.01)*
***A61P 27/02*** *(2006.01)* ***A61P 27/06*** *(2006.01)*
***C07D 293/06*** *(2006.01)*

(86) International application number:
**PCT/JP2009/053634**

(87) International publication number:
**WO 2009/107759 (03.09.2009 Gazette 2009/36)**

# (54) PROPHYLACTIC OR THERAPEUTIC AGENT FOR OCULAR DISEASE ACCOMPANIED BY OPTIC NERVE DISORDER

PROPHYLAKTIKUM ODER THERAPEUTIKUM FÜR AUGENERKRANKUNG MIT BEGLEITENDER SEHNERVSTÖRUNG

AGENT PROPHYLACTIQUE OU THÉRAPEUTIQUE POUR UNE MALADIE OCULAIRE ACCOMPAGNÉE D'UN TROUBLE DU NERF OPTIQUE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **28.02.2008 JP 2008047099**

(43) Date of publication of application:
**17.11.2010 Bulletin 2010/46**

(73) Proprietor: **Santen Pharmaceutical Co., Ltd**
**Osaka-shi**
**Osaka 533-8651 (JP)**

(72) Inventors:
- **HIRAI, Shin-ichiro**
**Ikoma-shi**
**Nara 630-0101 (JP)**
- **SASAOKA, Masaaki**
**Ikoma-shi**
**Nara 630-0101 (JP)**
- **SEIKE, Hisayuki**
**Ikoma-shi**
**Nara 630-0101 (JP)**

(74) Representative: **Müller-Boré & Partner**
**Patentanwälte PartG mbB**
**Friedenheimer Brücke 21**
**80639 München (DE)**

(56) References cited:
**EP-A1- 2 163 548** **WO-A1-2008/146721**
**JP-A- 62 294 613** **JP-A- 2008 013 448**
**JP-T- 2004 512 287** **US-A1- 2003 216 290**

- **GIRKIN, C. A.: 'Neuroprotection: Does it Work for All Neurological Diseases?' CLINICAL & SURGICAL JOURNAL OF OPHTHALMOLOGY vol. 24, no. 9, 2006, pages 362 - 367, XP008139859**
- **GABRYEL, B.: 'Ebselen attenuates oxidative stress in ischemic astrocytes depleted of glutathione.' COMPARISON WITH GLUTATHIONE PRECURSORS, PHARMACOLOGICAL REPORTS vol. 58, 2006, pages 381 - 392, XP008142268**
- **LIU, Q.: 'Oxidative Stress Is an Early Event in Hydrostatic Pressure-Induced Retinal Ganglion Cell Damage' INVESTIGATIVE OPHTHALMOLOGY & VISUAL SCIENCE vol. 48, no. 10, 2007, pages 4580 - 4589, XP008139942**
- **IZZOTTI, A.: 'The role of oxidative stress in glaucoma' MUTATION RESEARCH vol. 612, 2006, pages 105 - 114, XP025175775**
- **MAYUMI OKADA: 'Ryokunaisho o Meguru Wadai III. Ryokunaisho no Chiryo Shinkei Hogo to Yakubutsu Ryoho' KOWA IHO vol. 43, no. 2, 2000, pages 13 - 18, XP001525600**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 251 009 B1

- CALANDRELLA, N.: 'Degenerative and apoptotic events at retinal and optic nerve level after experimental induction of ocular hypertension' MOLECULAR AND CELLULAR BIOCHEMISTRY vol. 301, no. 1-2, 2007, pages 155 - 163, XP019506158
- HERIN, G. A.: 'The neuroprotective agent ebselen modifies NMDA receptor function via the redox modulatory site' JOURNAL OF NEUROCHEMISTRY vol. 78, no. 6, 2001, pages 1307 - 1314, XP008139943

## Description

The present invention relates to 2-phenyl-1,2-benzisoselenazol-3 (2H) -one or a salt thereof for use in a prophylactic or therapeutic treatment for an eye disease accompanied by optic nerve disorder, wherein the eye disease is glaucoma, glaucomatous optic neuropathy, glaucomatous visual field constriction, or glaucomatous optic atrophy.

The retina has a function to receive light from outside, and plays an important role for visual function. The retina is structurally a tissue having a thickness of from 0.1 to 0.5 mm which is composed of 10 layers such as retinal pigment epithelium layer, inner plexiform layer, ganglion cell layer, and nerve fiber layer. In the inner plexiform layer, there exist neurons called amacrine cells which are paired with ganglion cell neurites to form synapses. Since these neurons respond well both at the start and at the end of light irradiation, they are considered to work as a detector of light intensity. In the ganglion cell layer, there exist ganglion cells which are present in the innermost retina (hereinafter also referred to as "RGC") and are deeply involved in movement vision, peripheral vision, color vision, form vision, and the like. Further, in the nerve fiber layer, retinal vessels which are branches of the central artery and vein of the retina run and play a role of supplying retinal neurons with oxygen and nutrition.

When retinal vessels are occluded or narrowed because of a cause such as spasm, thrombus, or arteriosclerosis, disorder occurs in retinal blood circulation, and thus, the supply of oxygen and nutrition to the retina or optic nerve is blocked. Therefore, degeneration or loss of retinal neurons is caused, and optic nerve disorder is induced. Accordingly, the disorder of retinal blood circulation occupies especially an important position in retinal diseases. Examples of representative symptoms which are accompanied by the disorder of retinal blood circulation include retinal vascular occlusion in which retinal artery or retinal vein is occluded or narrowed (such as central retinal artery occlusion or branch retinal artery occlusion), diabetic retinopathy which is one of the causes of retinal detachment, and ischemic optic neuropathy in which visual dysfunction occurs. Further, it is considered that the ganglion cell death is deeply involved also in the incidence of macular degeneration, retinitis pigmentosa, Leber's disease, or the like.

Further, glaucoma is one of the eye diseases causing serious visual dysfunction which leads to visual loss if it is not appropriately treated. In glaucoma, among retinal neurons, particularly RGC is selectively damaged and optic nerve disorder is caused, which results in progressing to the visual field defect. Therefore, there has now been being established so-called "Neuroprotection", i.e., an idea that a prophylaxis of RGC disorder or a therapy in which RGC disorder is suppressed to the minimum will lead to an ultimate treatment of glaucoma (Non-patent document 1).

The detailed mechanism of glaucomatous optic nerve disorder has not been elucidated yet. However, a mechanical damage theory, in which optic nerve atrophy is caused by the direct compression of the optic nerve due to an increase in the intraocular pressure, and a circulatory disorder theory, in which circulatory disorder in the optic disc is the main cause of optic nerve atrophy, have been proposed, and it is considered that both of these mechanisms based on a mechanical damage and a circulatory disorder are related in a complex manner. Further, both the mechanical damage and the circulatory disorder cause an optic nerve axonal transport disorder, and it is considered that the disruption of supply of a neurotrophic factor accompanying this axonal transport disorder is one of the causes of RGC disorder (Non-patent document 2). In addition, glutamate is one of the neurotransmitters in the retina, however, it is considered that the excess activation of the glutamate signaling cascade by any cause is also one of the causes of RGC disorder (Non-patent document 3).

Accordingly, if there is a drug that has a protective effect on retinal neurons such as RGC, it is expected to be useful for prophylaxis or therapy of an eye disease accompanied by optic nerve disorder such as glaucoma, glaucomatous optic neuropathy, glaucomatous visual field constriction, glaucomatous optic atrophy, retinal vascular occlusion, retinitis pigmentosa, or Leber's disease.

On the other hand, 2-phenyl-1,2-benzisoselenazol-3(2H)-one (generic name: Ebselen, hereinafter also referred to as "Ebselen") has an antioxidative effect, and is reported to be useful for cerebral arteriosclerosis and chronic cerebral circulatory failure, and to activate glutamate transport in motor neurons (Non-patent document 4 and Patent documents 1 and 2). Further, Ebselen is reported to be useful for a keratoconjunctival disorder such as dry eye or superficial punctate keratopathy (Patent document 3).

As a report of study of the pharmacological effect of Ebselen on retinal cells, Non-patent document 5 reports that, in an ischemia/reperfusion rabbit model, Ebselen attenuated the reduction of the b-wave of the electroretinogram (ERG) after ischemia/reperfusion. This report only reports the effect of Ebselen on photoreceptor cells (visual cells) located on the outer side of the retinal tissue (on the side of the choroidal membrane tissue), and it does not describe or suggest at all the pharmacological effect of Ebselen on RGC located on the inner side of the retinal tissue (on the side of the vitreous body). RGC constitutes only a small portion of the retinal tissue, in other words, it makes up only about 1% of the neurons constituting the retina. Further, the retinal tissue is supplied with blood from two vascular systems. Photoreceptor cells are supplied with blood from the choroidal capillaries, and RGC is supplied with blood from the capillaries from the central retinal artery. Also from these points of view, it is considered that Non-patent document 5 only studies the effect of Ebselen on the outside layer of the retinal tissue, and does not study the pharmacological effect of Ebselen on RGC.

Further, Non-patent document 6 reports that Ebselen inhibited the cell death caused by glutamate excitotoxicity in chick retinal cells in an embryonic stage using the cells. As described above, RGC constitutes only a small portion of the retinal tissue, and therefore, what pharmacological effect on RGC Ebselen actually has remains unknown although the effect of Ebselen on the entire retinal tissue has been studied.

As described above, there is no report that the pharmacological effect of Ebselen on RGC is studied. Also, the above-mentioned prior art documents do not suggest the pharmacological effect of Ebselen on the prophylaxis or therapy of an eye disease accompanied by optic nerve disorder such as glaucoma, glaucomatous optic neuropathy, glaucomatous visual field constriction, glaucomatous optic atrophy, retinal vascular occlusion, retinitis pigmentosa, or Leber's disease.

Patent document 1: JP-A-2001-261555
Patent document 2: WO 2004/071419
Patent document 3: WO 2006/123676
Non-patent document 1: Ophthalmology, 40, 251-273, 1998
Non-patent document 2: Ophthalmology, 44, 1413-1416, 2002
Non-patent document 3: Surv. Ophthalmol., 48, S38-S46, 2003
Non-patent document 4: Proc. Natl. Acad. Sci. USA, 100(13), 7919-7924 (2003)
Non-patent document 5: Faseb Journal, 2004, 18, 4-5, abst. 429.13.
Non-patent document 6: Brain Research, 2005, 1039, 146-152

In short, it is an interesting subject to search a novel pharmaceutical use of Ebselen.

The present inventors made intensive studies to search a novel pharmaceutical use of Ebselen and found that, as described later, Ebselen increases the expression level of a gene of a phase II xenobiotic metabolizing enzyme or an antioxidant enzyme, which is inducibly regulated by Nrf2 (Nf-E2 related factor 2) in retinal neurons and suppresses a decrease in the number of cells in the RGC layer in a dose-dependent manner in an ischemia/reperfusion model which is widely used as a glaucoma model, and thus the present invention was achieved.

That is, the present invention is directed to a prophylactic or therapeutic agent for an eye disease accompanied by optic nerve disorder such as glaucoma, glaucomatous optic neuropathy, glaucomatous visual field constriction, or glucomatous optic atrophy, comprising Ebselen or a salt thereof as an active ingredient. In particular, the present invention is useful as a prophylactic or therapeutic agent for an eye disease accompanied by retinal ganglion cell disorder such as glaucoma, glaucomatous optic neuropathy, glaucomatous visual field constriction, or glaucomatous optic atrophy, in which RGC is selectively damaged and optic nerve disorder is caused.

When a test described below was performed, it was shown that Ebselen increases the expression levels of target genes whose expression is regulated by Nrf2, i.e., genes of glutamate-cysteine ligase, modifier subunit (hereinafter also referred to as "GCLM"), thioredoxin reductase (hereinafter also referred to as "TRxR"), Heme oxigenase 1 (hereinafter also referred to as "HO1"), and NAD(P)H quinone oxidoreductase 1 (hereinafter also referred to as "NQO1") in rat fetus-derived cultured retinal neuron death. That is, Ebselen has an ability to activate Nrf2 and inducibly regulates the expression of genes of a phase II xenobiotic metabolizing enzyme (for example, NQO1) and an antioxidant enzyme (for example, HO1) in rat fetus-derived cultured retinal neurons. Further, when the effect of Ebselen on RGC death induced by retinal ischemia/reperfusion described below was studied, Ebselen suppressed the RGC death.

Accordingly, Ebselen has an ability to activate Nrf2 and improves RGC damage by inducibly regulating the expression of genes of a phase II xenobiotic metabolizing enzyme and an antioxidant enzyme, and therefore is useful as a prophylactic or therapeutic agent for an eye disease accompanied by optic nerve disorder.

Ebselen serving as an active ingredient of the present invention is a condensed heterocyclic compound represented by the following chemical structural formula [I].

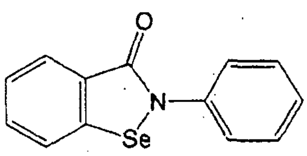

[I]

Further, the salt of Ebselen is not particularly limited as long as it is a pharmaceutically acceptable salt, and examples thereof include a salt with an inorganic acid such as hydrochloric acid, nitric acid, or sulfuric acid; and a salt with an organic acid such as acetic acid, fumaric acid, maleic acid, succinic acid, or tartaric acid. Ebselen may be in the form of a solvate.

In the present invention, the "eye disease accompanied by optic nerve disorder" include glaucoma, glaucomatous optic neuropathy, glaucomatous visual field constriction, and glaucomatous optic atrophy.

In the present invention, the term "retinal neurons" means neurons involved in transmission of visual signals

to the brain, and specifically means visual cells, horizontal cells, bipolar cells, retinal ganglion cells, amacrine cells, and the like.

In the present invention, the term "protection of retinal neurons" not only means suppression of retinal neuron death and/or retinal neuron hypofunction which occurs from any cause, but also means prophylaxis of retinal neuron death and/or retinal neuron hypofunction which may occur in future.

In the present invention, the term "protection of retinal ganglion cells" not only means suppression of retinal ganglion cell death and/or retinal ganglion cell hypofunction which occurs from any cause, but also means prophylaxis of retinal ganglion cell death and/or retinal ganglion cell hypofunction which may occur in future.

In the present invention, the term "retinal neuron death" means apoptosis and/or necrosis of retinal neurons, and particularly means apoptosis of retinal neurons.

In the present invention, the term "retinal ganglion cell death" means apoptosis and/or necrosis of retinal ganglion cells, and particularly means apoptosis of retinal ganglion cells.

Ebselen can be formulated into a single preparation or a combination preparation by adding a pharmaceutically acceptable additive as needed using a widely used technique.

When Ebselen is used for prophylaxis or therapy of the above-mentioned eye disease, it can be administered to a patient orally or parenterally. Examples of the route of administration include oral administration, topical administration to eyes (such as instillation administration, administration into conjunctival sac, intravitreal administration, subconjunctival administration, and sub-Tenon's administration), intravenous administration and transdermal administration. Further, it is formulated into a dosage form suitable for administration along with a pharmaceutically acceptable additive as needed. Examples of the dosage form suitable for oral administration include a tablet, a capsule, a granule, and a powder, and examples of the dosage form suitable for parenteral administration include injections, an eye drop, an ophthalmic ointment, a patch, a gel, and an insert. These can be prepared using a common technique widely used in this field. Further, Ebselen can also be formulated into a preparation for intraocular implant or a DDS (drug delivery system) preparation such as a microsphere other than those preparations.

For example, the tablet can be prepared by properly selecting and using a diluent such as lactose, glucose, D-mannitol, anhydrous calcium hydrogen phosphate, starch, or sucrose; a disintegrant such as carboxymethyl cellulose, calcium carboxymethyl cellulose, croscarmellose sodium, crosspovidone, starch, partially pregelatinized starch, or low-substituted hydroxypropyl cellulose; a binder such as hydroxypropyl cellulose, ethyl cellulose, gum arabic, starch, partially pregelatinized starch, polyvinyl pyrrolidone, or polyvinyl alcohol; a lubricant such as magnesium stearate, calcium stearate, talc, hydrous silicon dioxide, or a hydrogenated oil; a coating agent such as purified sucrose, hydroxypropyl methyl cellulose, hydroxypropyl cellulose, methyl cellulose, ethyl cellulose, or polyvinyl pyrrolidone; a flavoring substance such as citric acid, aspartame, ascorbic acid, or menthol; or the like.

The injection can be prepared by selecting and using a tonicity agent such as sodium chloride; a buffer such as sodium phosphate; a surfactant such as polyoxyethylene sorbitan monooleate; a viscous agent such as methyl cellulose; or the like as needed.

The eye drop can be prepared by selecting and using a tonicity agent such as sodium chloride or concentrated glycerin; a buffer such as sodium phosphate or sodium acetate; a surfactant such as polyoxyethylene sorbitan monooleate, polyoxyl 40 stearate, or polyoxyethylene hydrogenated castor oil; a stabilizer such as sodium citrate or sodium edetate; a preservative such as benzalkonium chloride or paraben; or the like as needed. The pH of the eye drop is permitted as long as it falls within the range that is acceptable as an ophthalmic preparation, but is generally preferably in the range of from 4 to 8. Further, the ophthalmic ointment can be prepared with a widely used base such as white petrolatum or liquid paraffin.

The insert can be prepared by pulverizing and mixing a biodegradable polymer such as hydroxypropyl cellulose, hydroxypropyl methyl cellulose, a carboxy vinyl polymer, or polyacrylic acid along with Ebselen and compression molding the resulting powder. If necessary, an excipient, a binder, a stabilizer, or a pH adjusting agent can be used. The preparation for intraocular implant can be prepared using a biodegradable polymer such as polylactic acid, polyglycolic acid, a lactic acid-glycolic acid copolymer, or hydroxypropyl cellulose.

The dose of Ebselen can be properly changed depending on the dosage form, severity of symptoms, age or body weight of a patient in need of administration, medical opinion, and the like. In the case of oral administration, it can be generally administered to an adult once or divided into several times at a dose of from 0.01 to 5000 mg, preferably from 0.1 to 2500 mg, more preferably from 0.5 to 1000 mg per day. In the case of an injection, it can be generally administered to an adult once or divided into several times at a dose of from 0.0001 to 2000 mg per day. In the case of an eye drop or an insert, generally a preparation containing the active ingredient in an amount of from 0.000001 to 10% (w/v), preferably from 0.00001 to 1% (w/v), more preferably from 0.0001 to 0.1% (w/v) can be administered once or several times per day. Further, in the case of a patch, a patch containing the active ingredient in an amount of from 0.0001 to 2000 mg can be applied to an adult, and in the case of a preparation for intraocular implant, a preparation for intraocular implant containing the active ingredient in an amount of from 0.0001 to 2000 mg can be implanted in an eye of an adult.

Hereinafter, the results of tests 1 and 2 and preparation examples will be shown. However, these examples are for understanding the present invention well, and are not meant to limit the scope of the present invention.

Examples

Test 1: Study of Ability to Induce Expression of Genes of Phase II Xenobiotic Metabolizing Enzyme and Antioxidant Enzyme using Rat Fetus-Derived Retinal Neuron Culture System

The ability of Ebselen to induce the expression of genes of a phase II xenobiotic metabolizing enzyme and an antioxidant enzyme was evaluated using a rat fetus-derived retinal neuron culture system (Brain Res. 2003; 967: 257-66). Incidentally, the rat fetus-derived retinal neuron culture system has been extensively used as a technique for culturing retinal neurons and has been widely used as one of the tools for studying optic nerve diseases such as glaucoma.

Evaluation Method

Retinal neurons of a rat fetus (Slc:Wistar/ST, 18 days old) were isolated and seeded onto a polyethyleneimine-coated plastic coverslip. Then, the cells were cultured for 3 days in an Eagle's minimum essential medium supplemented with 10% fetal bovine serum. Thereafter, a solvent (0.1% ethanol) or Ebselen was added thereto to give a final concentration of 25 μM, and the cells were incubated in the culture medium. Incidentally, the culture was performed under the conditions of 37°C and 5% $CO_2$. The culture medium was removed at each time point of 4, 8, or 12 hours after initiation of incubation. A mixed solution was prepared by adding 1% (v/v) β-mercaptoethanol (manufactured by BioRad, Cat No. 161-0710) to Buffer RLT attached to RNeasy Mini Kit (250) (manufactured by QIAGEN, Cat No. 74106), and the retinal neurons on the plastic coverslip were lysed with the thus prepared mixed solution. The total RNA was extracted from the resulting cell lysate according to the protocol attached to the RNeasy Mini Kit (250), and cDNA was synthesized using ExScript™ RT reagent Kit (manufactured by TAKARA, Cat No. RR035B). According to the protocol attached to SYBR Premix Ex Taq (Perfect Real Time) (manufactured by TAKARA, Cat No. RR041B), SYBR Premix Ex Taq (Perfect Real Time), ROX Reference Dye, the synthesized cDNA, and each primer pair of glyceraldehyde-3-phosphate dehydrogenase (hereinafter also referred to as "GAPDH"), GCLM, TRxR, HO-1, or NQO-1 (manufactured by Nihon Gene Research Laboratories Inc.) were mixed, and PCR reaction was performed using a quantitative PCR system (manufactured by Applied Biosystems, system name: ABI PRISM 7000), and the expression level of each of the genes of GAPDH, GCLM, TRxR, HO-1, and NQO-1 was measured. From the obtained results, a relative expression level of each target gene was calculated by normalizing the expression level of each target gene with the expression level of GAPDH which is a housekeeping gene according to the equation 1. Further, an induction ratio (%) of each target gene to the vehicle group was obtained by dividing the value of the expression level of each gene in the 25 μM Ebselen group by a mean value of the expression level of the same gene in the vehicle group according to the equation 2. Each data was shown as the mean ± standard deviation of three independent experiments.

Incidentally, the sequences of the respective primers used in this evaluation are as follows.

[Table 1]

| Name | Nucleotide sequence |
|---|---|
| GAPDH primer pair | GGGCTCATGACCACAGTCCA |
| | CACGCCACAGCTTTCCAGAG |
| GCLM primer pair | GTGTGATGCCACCAGATTTGAC |
| | CCTGGAAACTTGCCTCAGAGAG |
| TRxR primer pair | AATTTCCGGCAGTGTGTGTCC |
| | ACCCAAGAGCCATGCAATGAG |
| HO-1 primer pair | GTGCTCGCATGAACACTCTGG |
| | AGTGCCTGCAGCTCCTCAAAC |
| NQO-1 primer pair | GGAAGCTGCAGACCTGGTGATA |
| | GCATACGTGTAGGCGAATCCTG |

[Equation 1]

Relative expression level of each target gene = (Expression level of each gene / Expression level of GAPDH)

[Equation 2]

Induction ratio of each target gene to vehicle group (%) = (Expression level of each gene in 25 μM Ebselen group / Expression level of each gene in vehicle group)

(Results)

The results are shown in Table 2. As is apparent from Table 2, it was shown that Ebselen increases the expression levels of genes of GCLM, TRxR, HO-1, and NQO-1, which are a phase II xenobiotic metabolizing enzyme or an antioxidant enzyme in the rat fetus-derived retinal neuron culture system.

[Table 2]

| | Induction ratio of expression of each gene in 25 μM Ebselen group to vehicle group (%: mean ± standard deviation) | | |
|---|---|---|---|
| Name of each gene | After 4 hours | After 8 hours | After 12 hours |
| GCLM | 198 ± 18 | 194 ± 14 | 172 ± 13 |
| TRxR | 225 ± 11 | 193 ± 13 | 301 ± 26 |
| HO-1 | 1263 ± 365 | 1458 ± 307 | 1311 ± 237 |
| NQO-1 | 450 ± 74 | 937 ± 183 | 1266 ± 212 |

Test 2: Pharmacological Test using Rat Model of Ischemia/Reperfusion-Induced Retinal Damage

The usefulness of Ebselen against retinal ischemia/reperfusion damage was evaluated using a rat model of ischemia/reperfusion-induced retinal damage (Ophthalmologica. 1991; 203: 138-147) as a model for studying an effect on retinal damage induced by ischemia/reperfusion. Incidentally, the rat model of ischemia/reperfusion-induced retinal damage is an animal model in which retinal ischemia is induced by applying hydraulic pressure to the anterior segment of the eye, and after a given period of time, the blood flow was reperfused by releasing the hydraulic pressure, and is widely used as one of the animal models of optic nerve disorder mainly caused by glaucoma.

(Production Method for Rat Model of Ischemia/Reperfusion-Induced Retinal Damage and Evaluation Method)

A 1% (w/v) atropine sulfate hydrate ophthalmic solution (manufactured by Nihon Tenganyaku, Co. Ltd., product name: Nitten ATROPINE Ophthalmic Solution 1%) was instilled into the eye of a rat (Slc:SD, male, about 7 weeks old) to cause mydriasis, and general anesthesia was induced by allowing the rat to inhale a gas obtained by vaporizing 3% (v/v) halothane (manufactured by Takeda Pharmaceutical Company Limited., product name: Fluothane) by a mixed gas (2 L/min) of 100% (v/v) oxygen (0.5 L/min) and 100% (v/v) nitrous oxide (1.5 L/min), and the anesthesia was maintained under conditions of 1% (v/v) halothane. An infusion container containing physiological saline (manufactured by Otsuka Pharmaceutical Co., Ltd., product name: Otsuka Normal Saline) was hung from the ceiling, and a 30 G injection needle

connected to the container through a tube was inserted into the anterior chamber, and thus, a hydraulic pressure of 130 mmHg was applied to induce ischemia. 45 minutes later, the injection needle was removed, and retinal blood flow was reperfused. 7 days after the ischemia/reperfusion treatment, a pentobarbital sodium injection solution (manufactured by Dainippon Sumitomo Pharma Co., Ltd., product name: Nembutal injection solution) was intraperitoneally administered to the rat at a dose of 100 mg/kg to achieve general anesthesia, and the eyeball was enucleated. The enucleated eyeball was fixed by a mixed liquid of 25% (w/v) glutaraldehyde and 10% (w/v) neutral buffered formalin (glutaraldehyde : neutral buffered formalin = 1:9). The fixed eyeball was embedded in paraffin and sliced into sections, whereby retinal sections (thickness: 3 μm) were prepared. The sections were stained with hematoxylin-eosin. 8 retinal sections were prepared with an interval of 45 μm per one eye such that the optic nerve head was contained therein. From the 8 sections, 3 sections were arbitrarily selected, and with respect to the selected sections, a photograph of the retina in the range of from 1 to 1.5 mm on the left or right side from the optic nerve head was taken, and the number of cells in the retinal ganglion cell layer was measured. Thereafter, according to the equation 3, a suppression ratio (%) of Ebselen against a decrease in the number of cells in the retinal ganglion cell layer caused by ischemia/reperfusion damage was calculated such that the number of cells in the untreated group was taken as 100%, and the number of cells in the group in which the vehicle (a 1% (w/v) methyl cellulose solution) was orally administered to the rat subjected to the ischemia/reperfusion treatment was taken as 0%. Incidentally, the number of animals in each group is 6 to 8 (6 to 8 eyes).

[Equation 3]

$$\text{Suppression ratio against decrease in the number of cells in retinal ganglion cell layer (\%)} = (N_X - N_V) / (N_U - N_V) \times 100$$

$N_U$: The number of cells in the ganglion cell layer in the untreated group

$N_V$: The number of cells in the ganglion cell layer in the group in which the vehicle was orally administered to the rat subjected to the ischemia/reperfusion treatment

$N_X$: The number of cells in the ganglion cell layer in the group in which the drug was orally administered to the rat subjected to the ischemia/reperfusion treatment

(Administration method)

Vehicle administration group:

A 1% (w/v) methyl cellulose solution (prepared by dissolving methyl cellulose in purified water) was orally administered repeatedly twice daily for 12 days from 5 days before to 6 days after the date of ischemia/reperfusion treatment.

Ebselen administration group:

Ebselen suspended in a 1% (w/v) methyl cellulose solution was orally administered repeatedly twice daily for 12 days from 5 days before to 6 days after the date of ischemia/reperfusion treatment at a dose of 3 or 30 mg/kg.

(Results)

The results are shown in Table 3. As is apparent from Table 3, it was shown that Ebselen suppresses a decrease in the number of cells in the retinal ganglion cell layer in a dose-dependent manner in the rat model of ischemia/reperfusion-induced retinal damage.

[Table 3]

| Constitution of group | | Suppression ratio against decrease in the number of cells in retinal ganglion cell layer (%) |
|---|---|---|
| Ebselen | 3 mg/kg/day | 38.0 |
| | 30 mg/kg/day | 59.7 |

(Discussion)

From the above results, Ebselen increased the expression levels of genes of GCLM, TRxR, HO-1, and NQO-1, which are a phase II xenobiotic metabolizing enzyme or an antioxidant enzyme and are inducibly regulated by Nrf2 in retinal neurons, and also suppressed a decrease in the number of cells in the retinal ganglion cell layer in a dose-dependent manner in the ischemia/reperfusion model which is widely used as a glaucoma model. That is, it was shown that Ebselen has a remarkable prophylactic or improvement effect on an eye disease accompanied by optic nerve disorder such as glaucoma, glaucomatous optic neuropathy, glaucomatous visual field constriction, glaucomatous optic atrophy, optic nerve disorder caused by blood circulatory failure, ischemic optic nerve disorder, central retinal artery occlusion, branch retinal artery occlusion, central retinal vein occlusion, branch retinal vein occlusion, retinitis pigmentosa, Leber's disease, retinopathy of prematurity, retinal detachment, detachment of retinal pigment epithelium, macular degeneration, or diabetic retinopathy, in which retinal neuron death occurs. Further, it was shown that since Ebselen suppresses a decrease in the number of cells in the retinal ganglion cell layer in a dose-dependent manner, it has a particularly remarkable prophylactic or improvement effect on an eye disease accompanied by retinal ganglion cell disorder such as glaucoma, glaucomatous optic neuropathy, glaucomatous visual field constriction, or glaucomatous optic atrophy, in which RGC is selectively damaged.

[Preparation Examples]

The pharmaceuticals of the present invention will be more specifically described with reference to preparation examples, however, the present invention is not limited only to these preparation examples.

Formulation Example 1: Eye drop

| | |
|---|---|
| in 100 ml | |
| Ebselen | 10 mg |
| Sodium chloride | 900 mg |
| Polysorbate 80 | q.s. |
| Disodium hydrogen phosphate | q.s. |
| Sodium dihydrogen phosphate | q.s. |
| Sterile purified water | q.s. |

Ebselen and the other above-mentioned ingredients are added to sterile purified water, and these ingredients are mixed well, whereby an eye drop is prepared. By changing the addition amount of Ebselen, an eye drop containing Ebselen at a concentration of 0.05% (w/v), 0.1% (w/v), 0.5% (w/v), or 1% (w/v) can be prepared.

Formulation Example 2: Ophthalmic ointment

| | |
|---|---|
| in 100 g | |
| Ebselen | 0.3 g |
| Liquid paraffin | 10.0 g |
| White petrolatum | q.s. |

Ebselen is added to uniformly melted white petrolatum and liquid paraffin, these ingredients are mixed well, and the resulting mixture is gradually cooled, whereby an ophthalmic ointment is prepared. By changing the addition amount of Ebselen, an ophthalmic ointment containing Ebselen at a concentration of 0.05% (w/w), 0.1% (w/w), 0.5% (w/w), 1% (w/w), or 3% (w/w) can be prepared.

Formulation Example 3: Tablet

| | |
|---|---|
| in 100 mg | |
| Ebselen | 1 mg |
| Lactose | 66.4 mg |
| Cornstarch | 20 mg |
| Calcium carboxymethyl cellulose | 6 mg |
| Hydroxypropyl cellulose | 6 mg |
| Magnesium stearate | 0.6 mg |

Ebselen and lactose are mixed in a mixer, calcium carboxymethyl cellulose and hydroxypropyl cellulose are added thereto, and the resulting mixture is granulated. The obtained granules are dried, followed by sizing. Then, magnesium stearate is added and mixed with the sized granules and the resulting mixture is tableted with a tableting machine. By changing the addition amount of Ebselen, a tablet containing Ebselen in an amount of 0.1 mg, 10 mg, or 50 mg in 100 mg of tablet can be prepared.

Formulation Example 4: Injection

| | |
|---|---|
| in 10 ml | |
| Ebselen | 10 mg |
| Sodium chloride | 90 mg |
| Polysorbate 80 | q.s. |
| Sterile purified water | q.s. |

Ebselen and sodium chloride are dissolved in sterile purified water, whereby an injection is prepared. By changing the addition amount of Ebselen, an injection containing Ebselen in an amount of 0.1 mg, 10 mg, or 50 mg in 10 ml of injection can be prepared.

Ebselen has an ability to activate Nrf2 and inducibly regulates the expression of genes of a phase II xenobiotic metabolizing enzyme and an antioxidant enzyme, thereby improving RGC disorder. Therefore, Ebselen is useful as a prophylactic or therapeutic agent for an eye disease accompanied by optic nerve disorder.

SEQUENCE LISTING

<110> Santen Pharmaceutical

<120> Prophylactic or therapeutic agent vor disease accompanied
by optic disorder

<130> S10231

<140> EP 09714377.0
<141> 2009-02-27

<150> JP 2008-047099
<151> 2008-02-28

<160> 10

<170> PatentIn version 3.5

<210> 1
<211> 20
<212> DNA

<213> Artificial Sequence

<220>
<223> primer

<400> 1

gggctcatga ccacagtcca
20

<210> 2
<211> 20
<212> DNA
<213> Artificial Sequence

<220>
<223> primer

<400> 2

cacgccacag ctttccagag
20

<210> 3
<211> 22
<212> DNA
<213> Artificial Sequence

<220>

**Claims**

1. 2-phenyl-1,2-benzisoselenazol-3(2H)-one or a salt thereof for use in a prophylactic or therapeutic treatment for an eye disease accompanied by optic nerve disorder, wherein the eye disease is glaucoma, glaucomatous optic neuropathy, glaucomatous visual field constriction, or glaucomatous optic atrophy.

2. 2-phenyl-1,2-benzisoselenazol-3(2H)-one or a salt thereof for use according to claim 1, wherein the eye disease is glaucoma.

3. 2-phenyl-1,2-benzisoselenazol-3(2H)-one or a salt thereof for use according to claim 1 or 2, wherein the route of administration is instillation administration, intravitreal administration, subconjunctival administration, administration into conjunctival sac, sub-Tenon's administration, or oral administration.

4. The 2-phenyl-1,2-benzisoselenazol-3(2H)-one or a salt thereof for use according to claim 1 or 2, wherein the dosage form is an eye drop, an ophthalmic ointment, an insert, a patch, an injection, a tablet, a subtle granule, or a capsule.

**Patentansprüche**

1. 2-Phenyl-1,2-benzisoselenazol-3(2H)-on oder ein Salz davon zur Verwendung in einer prophylaktischen oder therapeutischen Behandlung für eine Augenkrankheit, begleitet von einer Sehnervstörung, wobei die Augenkrankheit ein Glaukom, eine glaukomatöse Sehnerverkrankung, eine glaukomatöse Sichtfeldverengung oder eine glaukomatöse Optikusatrophie ist.

2. 2-Phenyl-1,2-benzisoselenazol-3(2H)-on oder ein Salz davon zur Verwendung nach Anspruch 1, wobei die Augenkrankheit ein Glaukom ist.

**3.** 2-Phenyl-1,2-benzisoselenazol-3(2H)-on oder ein Salz davon zur Verwendung nach Anspruch 1 oder 2, wobei der Verabreichungsweg Verabreichung durch Einträufeln, intravitreale Verabreichung, subkonjunktivale Verabreichung, Verabreichung in den Bindehautsack, sub-Tenon-Verabreichung, oder orale Verabreichung ist.

**4.** 2-Phenyl-1,2-benzisoselenazol-3(2H)-on oder ein Salz davon zur Verwendung nach Anspruch 1 oder 2, wobei die Darreichungsform ein Augentropfen, eine Augensalbe, eine Einlage, eine Klappe, eine Injektion, eine Tablette, ein feines Granulat oder eine Kapsel ist.

**Revendications**

**1.** 2-Phényl-1,2-benzisosélénazol-3(2H)-one ou sel de celle-ci à utiliser dans un traitement prophylactique ou thérapeutique pour une maladie oculaire accompagnée d'un trouble du nerf optique, où la maladie oculaire est le glaucome, la neuropathie optique glaucomateuse, le rétrécissement glaucomateux du champ visuel, ou l'atrophie optique glaucomateuse.

**2.** 2-Phényl-1,2-benzisosélénazol-3(2H)-one ou sel de celle-ci à utiliser selon la revendication 1, où la maladie oculaire est le glaucome.

**3.** 2-Phényl-1,2-benzisosélénazol-3(2H)-one ou sel de celle-ci à utiliser selon la revendication 1 ou 2, où la voie d'administration est l'administration par instillation, l'administration intravitréenne, l'administration sous-conjonctivale, l'administration dans le cul-de-sac conjonctival, l'administration sous la capsule de Tenon ou l'administration orale.

**4.** 2-Phényl-1,2-benzisosélénazol-3(2H)-one ou sel de celle-ci à utiliser selon la revendication 1 ou 2, où la forme galénique est des gouttes oculaires, un onguent ophtalmique, un insert, un patch, une injection, un comprimé, un granulé fin ou une capsule.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001261555 A
- WO 2004071419 A
- WO 2006123676 A
- EP 09714377 A
- JP 2008047099 A


### Non-patent literature cited in the description


- *Ophthalmology,* 1998, vol. 40, 251-273
- *Ophthalmology,* 2002, vol. 44, 1413-1416
- *Surv. Ophthalmol.,* 2003, vol. 48, S38-S46
- *Proc. Natl. Acad. Sci. USA,* 2003, vol. 100 (13), 7919-7924
- *Faseb Journal,* 2004, vol. 18, 4-5
- *Brain Research,* 2005, vol. 1039, 146-152
- *Brain Res,* 2003, vol. 967, 257-66
- *Ophthalmologica,* 1991, vol. 203, 138-147